# EUROPEAN PATENT APPLICATION

(11) **EP 3 603 517 A1**
(43) Date of publication of application: **05.02.2020**
(21) Application number: 18770991.0
(22) Date of filing: 05.01.2018
(51) Int. Cl.: A61B 6/00

(54) **RADIOGRAPHY APPARATUS AND RADIOGRAPHY METHOD USING SAME**

(30) Priority: 20.03.2017 KR 20170034768
(71) Applicant: DRTECH CORP, Seongnam-Si, Gyeonggi-do 13216 (KR)
(72) Inventor: SHIN, Choul Woo, Seongnam-si Gyeonggi-do 13476 (KR); LEE, Jae Dong, Seongnam-si Gyeonggi-do 13434 (KR); YU, Byung Min, Seoul 02644 (KR)
(74) Representative: Michalski Hüttermann & Partner Patentanwälte mbB
(86) International application number: PCT/KR2018/000307
(87) International publication number: WO 2018/174389

(57) **Abstract**

The present invention relates to a radiography apparatus and a radiography method using the same and, more particularly, to a radiography apparatus for capturing an image of an object by using radiation, and a radiography method using the same. The radiography apparatus according to an embodiment of the present invention includes: a radiation emitting unit for emitting radiation to an object; a driving unit for moving the radiation emitting unit; a radiation detection unit for detecting radiation emitted from each of a plurality of imaging positions provided at each of imaging angle with respect to the object, so as to acquire a plurality of radiation images; and a plurality of radiation sources provided in the radiation emitting unit, such that, according to the movement of the radiation emitting unit, at least one thereof is arranged at one imaging position and at least one thereof is arranged at a position spaced apart from each imaging position.

## Description

### TECHNICAL FIELD

The present invention relates to a radiography apparatus and a radiography method using the same and, more particularly, to a radiography apparatus for capturing an image of an object by using radiation, and a radiography method using the same.

### BACKGROUND ART

Recently, as being grafted onto a semiconductor field, a radiography technology is rapidly evolving into a digital image technology having advantageous, such as a relatively high resolution, a wide dynamic region, an easy generation of electric signals, and convenient data processing and storage, instead of a traditional analogue method using a film. A digital-based image technology is strongly reflecting a clinical environmental demand that is an early diagnosis of a disease on the basis of an excellent diagnostic ability of a digital image.

Accordingly, there is introduced a digital mammography technology that is a breast-exclusive radiography technology which utilizes a unique biological tissue contrasting ability of the radiation and expresses the internal structure of a breast, as an object to be radiographed, with a high resolution image to detect lesions and microcalcification for an early diagnosis and detection of breast cancer. Such a digital mammography technology is being rapidly distributed due to the unique characteristics, such as enlarging an image, reducing the number of imaging times, enhancing a resolution, and minimizing exposure to radiation through a luminance and contrast ratio control, in addition to various advantages of the digital image technology.

Meanwhile, if an abnormal region (lesion) of an object is hidden by human tissues or the like, it is difficult to perform a diagnosis using a radiography apparatus which acquires a two-dimensional projected image. As a remedy for this problem, a technology of generating a three-dimensional image for a tested subject by capturing images of an object in various angles and synthesizing each of the images is being developed.

To this end, in a radiography apparatus used in a conventional digital breast tomosynthesis (DBT) system, radiation is emitted to an object while relatively rotating one radiation source with respect to the object to acquire radiation projected images in multiple directions, and a three-dimensional image is generated by synthesizing the images.

In such a conventional radiography apparatus, there occurs a motion blur phenomenon in which the boundary of an image acquired by a radiation detection unit is unclearly shown due to the movement of a radiation source, and the quality of the image is thus degraded. To prevent such a motion blur phenomenon, a stop-and-shoot method of capturing a projected image in a state where a radiation source is completely stopped state at an angle for imaging and then moving the radiation source to a next position for imaging is also used; however, since in this method, imaging should be performed in a state in which a radiation source is completely stopped, there is a problem in that an overall imaging time is delayed.

### (Related Art Document)

Japanese Patent Application Laid-open Publication No. 2011-125698

### DISCLOSURE OF THE INVENTION

### TECHNICAL PROBLEM

The present invention provides a radiography apparatus capable of acquiring a plurality of radiation images in various directions, and a radiography method using the same.

### TECHNICAL SOLUTION

A radiography apparatus according to an embodiment of the present invention includes: a radiation emitting unit for emitting radiation to an object; a driving unit for moving the radiation emitting unit; a radiation detection unit for detecting radiation emitted from each of a plurality of imaging positions provided at each of imaging angle with respect to the object, so as to acquire a plurality of radiation images; and a plurality of radiation sources provided in the radiation emitting unit, such that, according to the movement of the radiation emitting unit, at least one thereof is arranged at one imaging position and at least one thereof is arranged at a position spaced apart from each imaging position.

The radiation sources may be provided in the radiation emitting unit to have different intervals respectively from the adjacent imaging positions in one direction.

The radiation sources may be provided in the radiation emitting unit such that an interval therebetween is greater than each interval between the imaging positions.

The radiation sources may be sequenced in one direction, and the driving unit may move the radiation emitting unit along the sequenced direction of the radiation sources.

The radiation sources may be integrally moved with the radiation emitting unit while maintaining the interval therebetween.

The radiation sources may be sequentially activated according to the movement of the radiation emitting unit.

The radiation detection unit may acquire each radiation image during the movement of the radiation emitting unit.

The driving unit may change a moving speed of the radiation emitting unit according to an interval between each radiation source and each imaging position.

The radiography apparatus may further include a control unit for controlling an emitting direction of each radiation source according to the movement of the radiation emitting unit.

The control unit may control the emitting direction of the radiation sources, such that the emitting direction of each radiation source is towards the same position according to the movement of the radiation emitting unit.

The radiation detection unit may be provided to be rotatable according to the movement of the radiation emitting unit.

In addition, a radiography method according to another embodiment of the present invention include: acquiring a first radiation image by activating a first radiation source arranged at one imaging position among a plurality of radiation sources provided in a radiation emitting unit; moving the radiation emitting unit; and acquiring a second radiation image by activating a second radiation source arranged at one imaging position among the plurality of radiation sources provided in the radiation emitting unit.

In the moving of the radiation emitting unit, the radiation emitting unit may move in a distance shorter than each interval between the imaging positions.

In the moving of the radiation emitting unit, a moving speed of the radiation emitting unit may be changed according to an interval between each radiation source and each imaging position.

The acquiring of the first radiation image and the acquiring of the second radiation image may be performed while the radiation emitting unit moves.

The radiography method may further include changing an emitting direction of each radiation source, such that the emitting directions of the first radiation source and the second radiation source are towards the same position as a position before the radiation emitting unit moves.

The radiography method may further include the rotating of a radiation detection unit between the acquiring of the first radiation image and the acquiring of the second radiation image.

The acquiring of the first radiation image, the moving of the radiation emitting unit, and the acquiring of the second radiation image may be repeated until all radiation images are acquired at each imaging position.

The first radiation image may include a pre-shot image.

### ADVANTAGEOUS EFFECTS

According to the radiography apparatus and the radiography method using the same of embodiments of the present invention, since the radiation sources of which at least one is arranged at one imaging position and at least one is arranged at a position spaced apart from each imaging position captures the radiation projected image at the imaging positions according to the movement of the radiation emitting unit, the moving distance of the radiation emitting unit can be minimized, and an imaging time can thus be shortened.

In addition, since the radiation emitting unit, in which a plurality of radiation sources are provided, moves to acquire each radiation projected image at each of imaging position, the number of radiation sources can be reduced, and since a moving speed of the radiation emitting unit is reduced at the time of acquiring the radiation projected image, a motion blur phenomenon can be minimized.

Also, according to the radiography apparatus and the radiography method using the same of embodiments of the present invention, since a plurality of radiation sources provided in a radiation emitting unit are sequentially activated and captures a radiation projected image in various angles, each radiation projected image can be rapidly captured without considering a standby time to activate the radiation sources; therefore, a three-dimensional image of a high resolution can be acquired, and a lesion with respect to an object can be accurately diagnosed.

### BRIEF DESCRIPTION OF THE DRAWINGS

FIG. 1 is a drawing illustrating a digital breast tomosynthesis apparatus.
FIG. 2 is a drawing illustrating an aspect of acquiring a radiation projected image from a radiography apparatus.
FIG. 3 is a drawing schematically illustrating a radiography apparatus according to one embodiment of the present invention.
FIG. 4 to FIG. 7 are drawings illustrating aspects of acquiring radiation projected images according to one embodiment of the present invention.
FIG. 8 is a drawing schematically illustrating a radiography apparatus according to another embodiment of the present invention.
FIG. 9 is a drawing schematically illustrating a radiography method according to an embodiment of the present invention.

### MODE FOR CARRYING OUT THE INVENTION

Hereinafter, embodiments of the present invention will be described in detail with reference to the accompanying drawings. The present invention may, however, be embodied in different various forms without being limited to the embodiments set forth hereinafter. Rather, these embodiments are provided so that this disclosure of the present invention will be complete, and will fully convey the scope of the present invention to those skilled in the art. In the drawings, like reference numerals refer to like elements throughout.

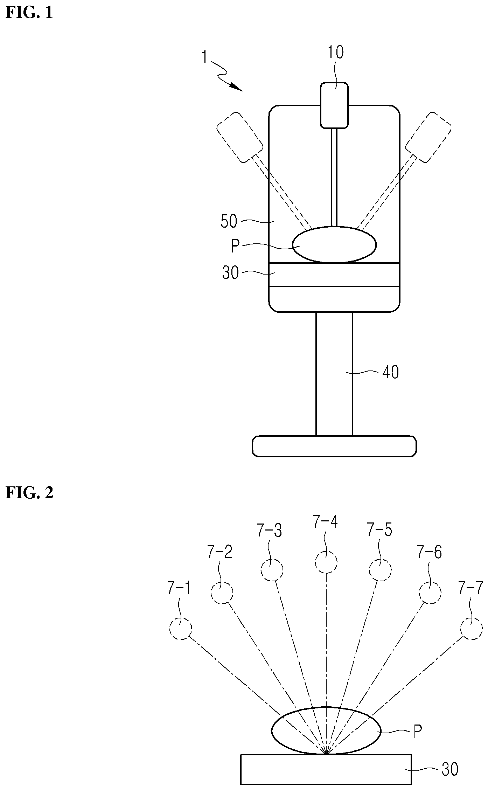

FIG. 1 is a drawing illustrating a digital breast tomosynthesis apparatus, and FIG. 2 is a drawing illustrating an aspect of acquiring a radiation image from a radiography apparatus.

When referring to FIG. 1 and FIG. 2, the digital breast tomosynthesis (DBT) apparatus (1) includes a support (40) having a lower end part fixed to the floor, a main body (50) provided to be able to ascend and descend along the support (40), a radiation detection unit (30) provided in the lower part of the main body (50), and a radiation emitting unit (10) provided in the upper part of the main body (50).

When a testee is positioned for imaging, in the digital breast tomosynthesis apparatus (1), the main body (50) ascends or descends along the support (40) to adjust a height, such that an object to be imaged (for example, breast) (P) of the testee is put on the radiation detection unit (30). Next, the radiation emitting unit (10) is rotated along a plurality of imaging positions disposed at each of imaging angle with respect to the object to be imaged, and a radiation source provided in the radiation emitting unit (10) images the object to be imaged while passing each imaging position at a constant speed according to the movement of the radiation emitting unit (10). At this time, either a stop-and-shot method or a continuous shot method may be used, wherein: in the stop-and-shot method, a radiation projected image is captured in a state of stopping the movement of the radiation emitting unit (10) once the radiation source is moved to an imaging position, and another radiation projected image is captured by moving the radiation source to a next imaging position; and in the continuous shot method, a radiation projected image is captured in a very short time at an imaging position during the movement of the radiation emitting unit (10), and another radiation projected image is captured by moving the radiation source to a next imaging position.

Here, a radiography apparatus used in a conventional digital breast tomosynthesis apparatus (1) acquired a radiation projected image by relatively rotating one radiation source with respect to an object. That is, for example, when one radiation source captures radiation projected images at an N number of imaging positions, that is, seven imaging positions provided at each of imaging angle, the radiation source performs imaging at each of imaging positions of 7-1, 7-2, ..., 7-7 to acquire a projected image.

However, in such a radiography apparatus, there occurs a motion blur phenomenon in which the boundary of an image acquired by a radiation detection unit is unclearly shown due to the movement of a radiation source, and the quality of the image is thus degraded. In addition, when a projected image is captured in a state where the radiation source is completely stopped while being relatively rotated with respect to an object, imaging should be performed in a state in which the radiation source is completely stopped in every position, and an overall imaging time is thus delayed.

To solve these problems, although not illustrated, the radiography apparatus can acquire a radiation projected image by a plurality of radiation sources fixed at each imaging position with respect to the object (P). In this case, an N number of radiation sources are provided, and each is fixed, and arranged, for example, at positions of 7-1, 7-2, ..., 7-7 at each of imaging angle, such that a radiation projected image is acquired at each position.

In this case, the radiography apparatus can prevent a motion blur phenomenon. However, as multiple radiation sources fixed and arranged in every imaging angle are used, product costs are increased, and maintenance costs are thereby increased. In addition, as many radiation sources are arranged, arrangement intervals are narrowed, and the radiation sources are thus difficult to be provided in a desired arrangement.

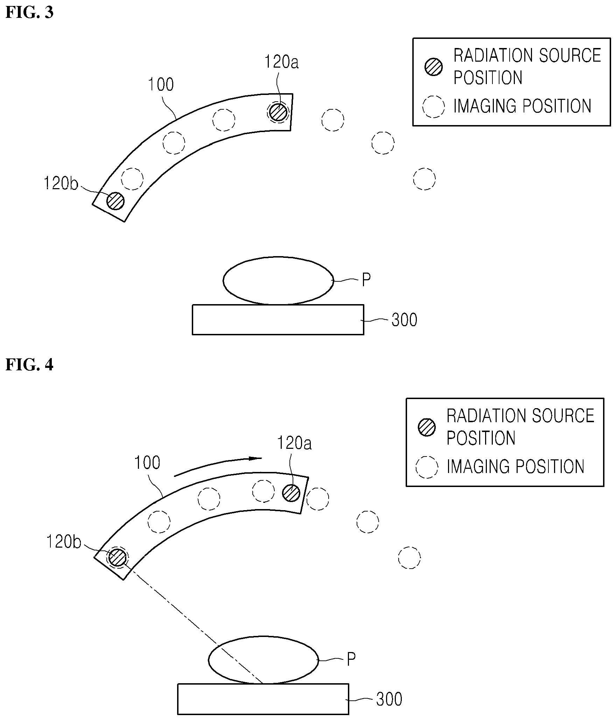

FIG. 3 is a drawing schematically illustrating a radiography apparatus according to an embodiment of the present invention, and FIG. 4 to FIG. 7 are drawings illustrating aspects of acquiring radiation projected images according to one embodiment of the present invention.

When referring to FIG. 3 to FIG. 7, a radiography apparatus according to an embodiment of the present invention includes: a radiation emitting unit (100) for emitting radiation to the object (P); a driving unit (not illustrated) for moving the radiation emitting unit (100); a radiation detection unit (300) for detecting radiation emitted from each of a plurality of imaging positions provided at each of imaging angle with respect to the object (P) so as to acquire a plurality of radiation images; and a plurality of radiation sources (120a, 120b) provided in the radiation emitting unit, such that at least one thereof is arranged at one imaging position and at least one thereof is arranged at a position spaced apart from each imaging position, according to the movement of the radiation emitting unit (100).

Here, the radiation sources (120a, 120b) are plurally provided in the radiation emitting unit (100), and the radiation emitting unit (100) moves along an sequenced direction of the radiation sources (120) by the driving unit, such that each radiation projected images is acquired before, after, or during the movement of the radiation emitting unit (100).

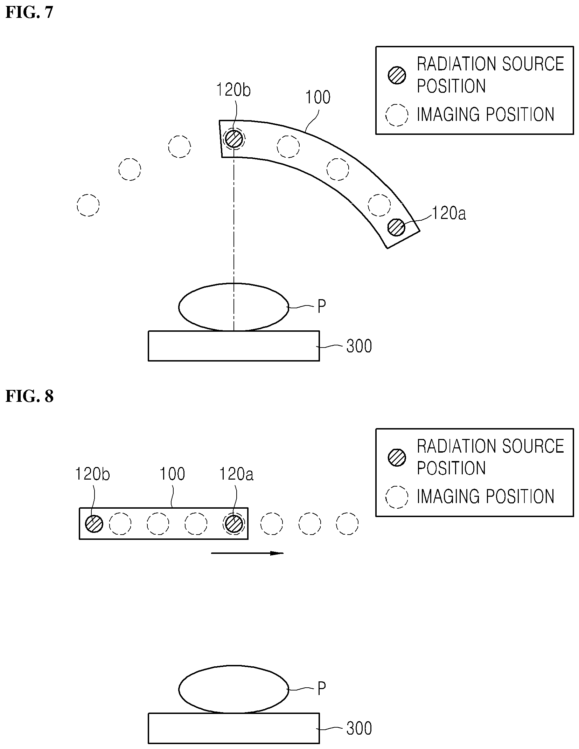

Regarding FIG. 3 to FIG. 7, one embodiment in which each imaging position is sequenced along the shape of an arc will be explained, and regarding FIG. 8, another embodiment in which each imaging position is sequenced along the shape of a straight line will be explained, wherein the sequenced direction of imaging positions is not limited thereto, and embodiments may be of course applied to all cases in which imaging positions are sequenced in one direction.

In addition, in embodiments explained hereinafter, radiation signifies not only X rays but also electromagnetic waves including α rays, β rays, γ rays, and the like, and the object (P) to which the radiation is emitted may be a human breast, but is not limited thereto.

The radiation sources (120a, 120b) emit radiation. To this end, the radiation sources (120) may generate radiation by emitting electron beams to a target, and may include a field emission electrode that generates electrons in an emitter electrode by applying an electric field. Here, as the field emission electrode, an electric field emission electrode including a protruding sharp end may be used and configured to easily emit electrons even when applying a small electric field, and a carbon nano-tube (CNT) having a very high field enhancement factor by having a geometrical structure with a low work function and a high aspect ratio may be used as the sharp end of the electric field emission electrode.

In addition, the radiation sources (120a, 120b) may include a thermoelectron emission electrode that generates electrons by heating a filament. In this case, as the filament is heated by electric power applied to the filament, thermoelectrons are generated, and the thermoelectrons collide with a target to generate radiation.

The radiation sources (120a, 120b) are plurally provided in the radiation emitting unit (100), such that at least one thereof is arranged at one imaging position and at least one thereof is arranged at a position spaced apart from each imaging position. In this case, each of the radiation sources (120a, 120b) may be provided in the radiation emitting unit (100) to have different intervals respectively from the adjacent imaging positions in one direction. FIG. 3 to FIG. 7 illustrate embodiments in which two radiation sources (120a, 120b) are provided, but two or more of various number of radiation sources may be of course provided in the radiation emitting unit (100). The radiation sources (120a, 120b) respectively emit radiation toward an emission position, for example, the central part of the radiation detection unit (300), and radiation emitted from the radiation sources (120) is emitted to the object (P), for example, a breast, positioned on the radiation detection unit (300).

The radiation sources (120a, 120b) according to one embodiment of the present invention are plurally provided, but are provided by a number smaller than the number of projected images required to synthesize a three-dimensional image. When radiation projected images captured at an N number of positions are required, for example, radiation projected images are required to be captured at seven positions, at each of imaging angle to synthesize a three-dimensional image, an NA number of radiation sources according to one embodiment of the present invention may be provided in, for example, two or three to six radiation sources may be provided. Likewise, by reducing the number of the radiation sources to the number smaller than the required number of radiation projected images to synthesize a three-dimensional image, a space to provide the radiation sources (120) is secured, and a reduction in product costs and ease of maintenance can be achieved.

The radiation sources (120a, 120b) may be provided by being sequenced in the radiation emitting unit in a direction along each imaging position. That is, the radiation sources (120a, 120b) may be sequenced along the shape of an arc as illustrated in FIG. 3 to FIG. 7, and when each radiation source (120a, 120b) is sequenced along the shape of an arc, a distance from the radiation detection unit (300) according to the emitting direction of the radiation sources (120a, 120b), that is, an interval between each radiation source (120a, 120b) and the central part of the radiation detection unit (300) which corresponds to a penetration position that is a position on the radiation detection unit (300) that the radiation sources (120a, 120b) reach by being extended in the emitting direction, may become maintained identically per radiation source (120), such that each radiation source (120a, 120b) may be incident to an object with uniform strength. Here, the emitting direction signifies a direction along the central line of radiation emitted from the radiation sources (120).

A plurality of radiation sources (120a, 120b) are respectively provided in the radiation emitting unit (100). As described hereinafter, the radiation emitting unit (100) moves along an imaging position, that is, the shape of an arc, by the driving unit. In the drawings, the radiation emitting unit (100) has the same shape as the sequenced direction of the radiation sources (120a, 120b), that is, the shape of an arc, but the radiation emitting unit (100) may be of course provided with a plurality of radiation sources (120a, 120b) and has various shapes capable of supporting the radiation sources.

The plurality of radiation sources (120a, 120b) integrally move with the radiation emitting unit (100) while an interval therebetween is maintained according to the movement of the radiation emitting unit (100). That is, the plurality of radiation sources (120a, 120b) are provided at constant intervals in the radiation emitting unit (100), and the plurality of radiation sources (120a, 120b) integrally move while maintaining constant intervals, as the radiation emitting unit (100) moves by the driving unit. A procedure of acquiring a radiation projected image as the radiation sources (120a, 120b) move integrally with the radiation emitting unit (100) will be described regarding an operating procedure of the driving unit later.

The driving unit moves the radiation emitting units (120a, 120b) according to each imaging position. As illustrated in FIG. 3 to FIG. 7, when each imaging position is sequenced along the shape of an arc, the driving unit moves the radiation emitting units (120a, 120b) along the shape of an arc. The driving unit may move the radiation emitting unit (100) by using a motor, an electromagnet, or the like. To easily control the moving direction of the radiation emitting unit (100), the radiography apparatus according to one embodiment of the present invention further includes a support unit (not illustrated) having the radiation emitting unit (100) seated thereon, and the driving unit may move the radiation emitting unit (100) on the support unit. In this case, a guide (not illustrated) extended along the moving path of the radiation emitting unit (100) may be produced in the support unit, and the radiation emitting unit (100) may be moved along the guide by being coupled with the guide provided in the support unit. Such a guide may be formed of a linear motion (LM) guide, a rail, or the like capable of moving the radiation emitting unit (100) along the moving path, and a bearing or the like may be of course provided along the guide to reduce resistance against the radiation emitting unit (100) coupled to the guide.

The radiation detection unit (300) detects radiation penetrating an object by being respectively emitted from a plurality of imaging positions provided at each of imaging angle with respect to the object and acquires a plurality of radiation images, that is, radiation projected images. The radiation detection unit (300) may include a digital-type radiation detection unit (300) using a thin film transistor.

Here, the radiation detection unit (300) may be provided to be rotatable according to the movement of the radiation emitting unit (100). Accordingly, the radiation detection unit (300) may be rotated to face each imaging position among a plurality of imaging positions.

When a plurality of radiation projected images are acquired from a plurality of imaging positions provided at each of imaging angle with respect to an object, the emitting angle of radiation at imaging positions arranged at both ends is greatly deviated from the central axis direction of the radiation detection unit (300). Accordingly, radiation projected images captured at the imaging positions arranged at both ends had a problem in which distortion of an image increases, and in order to solve the problem, a separate image processing or the like needed to be performed.

Therefore, the radiography apparatus according to an embodiment of the present invention may maintain radiation emitted from each imaging position to be maximally emitted to the radiation detection unit (300) by rotating the radiation detection unit (300) along the sequenced direction of the radiation sources and thereby arranging the radiation detection to face each imaging position. Such a rotation of the radiation detection unit (300) may be performed in various manners in which the radiation detection unit (300) is directly rotated by a separate driving means or the radiation detection unit (300) is rotated in connection with the above-described driving unit.

Hereinafter, explanations will be made by exemplifying a case of requiring radiation projected images captured at seven imaging positions (see FIG. 2) at each of imaging angle to synthesize a three-dimensional image according to the movement of the radiation emitting unit in accordance with the operation of the driving unit.

First, the radiography apparatus according to one embodiment of the present invention may be arranged as illustrated in FIG. 3 before the radiation emitting unit (100) moves. That is, before the radiation emitting unit (100) moves, the first radiation source (120a) may be arranged at the imaging position (7-4) in a direction perpendicular to the radiation detection unit (30), and the second radiation source (120b) may be arranged at a position spaced apart from each imaging position (7-1, 7-2, ..., 7-7), for example, the left side of the imaging position (7-1). In this case, before the radiation emitting unit (100) moves, the first radiation source (120a) may be activated and capture a free-shot image for determining an imaging condition such as an exposure amount of radiation amount. Here, the value of the amount of radiation emitted from the first radiation source (120a) to capture a pre-shot image may be different from the value of the amount of radiation emitted from each radiation source (120a, 120b) to capture a radiation projected image at each imaging position (7-1, 7-2, ..., 7-7). That is, a pre-shot image may be captured by radiation having a radiation amount different from that for a radiation projected image. Likewise, before the radiation emitting unit (100) moves, the first radiation source (120a) is arranged in a direction perpendicular to the radiation detection unit (30), such that a pre-shot image that is a radiation image for determining an imaging condition such as an exposure amount of radiation can be easily captured before capturing a radiation projected image.

After a pre-shot image is captured or when a pre-shot image needs not to be captured, a radiation projected image is captured according to procedures illustrated in FIG. 4 to FIG. 7. That is, as described above, according to the movement of the radiation emitting unit (100), a plurality of radiation projected images are captured at each imaging position by sequentially activating a plurality of radiation sources provided in the radiation emitting unit (100) in a manner that at least one radiation source is arranged at an imaging position and at least one radiation source is arranged at a position spaced apart from the imaging position.

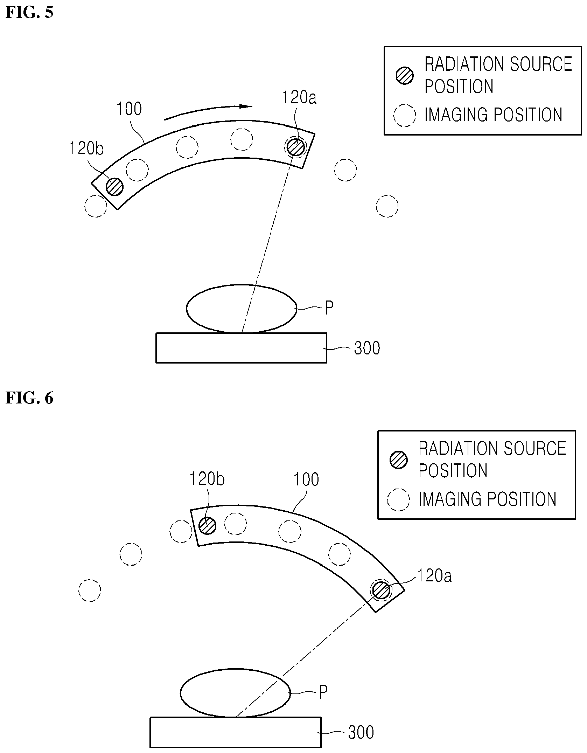

To explain in more detail, when the radiation emitting unit (100) moves in one direction (right direction in the drawings) along the sequenced direction of the imaging positions, the second radiation source (120b) is arranged at one imaging position (7-1), and the first radiation source (120a) is arranged (FIG. 4) at a position deviated from the imaging position by being spaced apart from each imaging position. Here, the second radiation source (120b) is activated at one imaging position (7-1), and captures a first radiation projected image. In addition, when the radiation emitting unit (100) moves in the sequenced direction of the imaging positions, the first radiation source (120a) is arranged at one imaging position (7-5), and the second radiation source (120b) is arranged (FIG. 5) at a position spaced apart from each imaging position. Here, the first radiation source (120a) is activated at one imaging position (7-5), and captures a second radiation projected image. Through such a procedure, third to fifth radiation projected images are sequentially captured. Also, according to the movement of the radiation emitting unit (100), the first radiation source (120a) is arranged at one imaging position (7-7) and captures a sixth radiation projected image (FIG. 6), and the second radiation source (120b) is arranged at one imaging position (7-4) and captures a seventh radiation projected image (FIG. 7). Then, all radiation projected images can be acquired in each imaging position (7-1, 7-2, ..., 7-7). Here, as described above, the seventh radiation projected image captured by the second radiation source (120b) at the imaging position (7-4) may be captured with a radiation amount different from the radiation amount emitted from the first radiation source (120a) when capturing a pre-shot image.

In the procedure above, as for the radiation sources (120a, 120b), the second radiation source (120b) is arranged at a position spaced apart from each imaging position when the first radiation source (120a) is arranged at one imaging position, and the first radiation source (120a) is arranged at a position spaced apart from each imaging position when the second radiation source (120b) is arranged at one imaging position according to the movement of the radiation emitting unit (100). In addition, the first radiation source (120a) and the second radiation source (120b) have different intervals from imaging positions respectively adjacent in one direction, and are integrally moved according to the movement of the radiation emitting unit (100).

In this case, the moving distance of the radiation sources may be minimized by being reduced to almost a half compared to when capturing a radiation projected image by relatively rotating one radiation source with respect to an object (P). Also, when capturing a radiation projected image according to each imaging position during the same time period, a moving speed of the radiation sources may be reduced to almost a half, and a motion blur phenomenon according to the movement of the radiation sources can be thus prevented.

In addition, by capturing a radiation projected image at various angles by sequentially activating each radiation source provided in the radiation emitting unit (100), after the radiation source (120a) is activated and captures a radiation projected image, the second radiation source (120b) can promptly capture a radiation projected image without considering an activation standby time to prepare for capturing of a radiation projected image.

Here, the radiation emitting unit (100) may capture a radiation projected image according to each imaging position while reciprocating in one direction and a different direction opposed to the one direction. However, if the radiation sources (120a, 120b) are arranged in the radiation emitting unit such that an interval therebetween is greater than intervals between the imaging positions, a radiation projected image may be acquired at all imaging positions even when the radiation emitting unit moves only in one direction.

Likewise, as a procedure of capturing a radiation projected image, either a stop-and-shot method or a continuous shot method may be used, wherein: in the stop-and-shot method, a radiation projected image is captured by stopping the movement of the radiation emitting unit (100) once the radiation sources (120a, 120b) are moved to each imaging position, and another radiation projected image is captured by moving the radiation sources to a next imaging position; and in the continuous shot method, a radiation projected image is captured by activating each radiation source (120a, 120b) during the movement of the radiation emitting unit (100), and another radiation projected image is captured by moving the radiation sources to a next imaging position.

Here, the radiography apparatus according to an embodiment of the present invention can reduce an imaging time by minimizing the moving distance of the radiation emitting unit (100) in the stop-and-shot method of capturing a radiation projected image by stopping the movement of the radiation emitting unit (100), and can minimize a motion blur phenomenon by reducing a moving speed of the radiation sources in the continuous shot method of capturing a radiation projected image during the movement of the radiation emitting unit (100).

In addition, the driving unit may change a moving speed of the radiation emitting unit (100) according to an interval between a radiation source and an imaging position. That is, in the continuous shot method of capturing a radiation projected image during the movement of the radiation emitting unit (100), the driving unit minimizes a motion blur phenomenon by reducing a moving speed of the radiation emitting unit (100) when the radiation emitting unit (100) moves such that one radiation source becomes adjacent to an imaging position within a predetermined interval. Moreover, when each radiation source is spaced apart from an imaging position by a predetermined interval or longer, a moving speed of the radiation emitting unit (100) may be changed to shorten an imaging time by increasing a moving speed of the radiation emitting unit (100).

Explanations were made above by exemplifying a case in which the number (NA) of radiation sources is two, but the number (NA) of radiation sources may be of course various other numbers such as three and four. In this case, by increasing the number (NA) of radiation sources, the moving distance of the radiation emitting unit (100) and an overall imaging period may be of course proportionally reduced.

FIG. 8 is a drawing schematically illustrating a radiography apparatus according to another embodiment of the present invention. Only an imaging position and a radiation source sequenced direction of the radiography apparatus according to another embodiment of the present invention in FIG. 8 are different from those of the radiography apparatus according to one embodiment of the present invention in FIG. 3, and repeated explanations regarding the radiography apparatus according to one embodiment of the present invention will thus be omitted.

The radiation sources (120a, 120b) may be provided by being sequenced in the radiation emitting unit (100) along the shape of a straight line.

When the radiation emitting unit (100) moves in the straight line direction along imaging positions sequenced in the shape of a straight line, the second radiation source (120b) is arranged at one imaging position (7-1), and the first radiation source (120a) is arranged at a position deviated from the imaging positions by being spaced apart from each imaging position. Here, the second radiation source (120b) is activated at one imaging position (7-1), and captures a first radiation projected image. In addition, when the radiation emitting unit (100) moves in the sequenced direction of imaging positions, the first radiation source (120a) is arranged at one imaging position (7-5), and the second radiation source (120b) is arranged at a position spaced apart from each imaging position. Here, the first radiation source (120a) is activated at one imaging position (7-5), and captures a second radiation projected image. Through such a procedure, third to fifth radiation projected images are sequentially captured. Also, according to the movement of the radiation emitting unit (100), when the first radiation source (120a) captures a sixth radiation projected image by being arranged at one imaging position (7-7), and the second radiation source (120b) captures a seventh radiation projected image is captured by being arranged at one imaging position (7-4), all radiation projected images can be acquired at each imaging position (7-1, 7-2, ..., 7-7). Moreover, as described above, the second radiation source (120b) may be arranged at one imaging position (7-4) perpendicular to the radiation detection unit (300) before the radiation emitting unit (100) moves. In this case, a pre-shot image may be captured before the radiation emitting unit (100) moves, and the pre-shot image and the seventh radiation projected image may be captured with radiation amounts different from each other as described above.

Here, when the imaging position and the radiation source are sequenced in the shape of an arc based on a penetration position as the center, distances from the radiation detection unit (300) according to the emitting directions of the radiation source before and after the movement of the radiation emitting unit (100) are identically maintained. However, when a plurality of radiation sources are sequenced in the shape of a straight line, a penetration position of the radiation sources is changed according to the movement of the radiation emitting unit (100). When the penetration position is changed, an acquired radiation projected image may be image-processed and then calibrated. However, the radiography apparatus according to another embodiment of the present invention further includes a control unit (not illustrated) for controlling the emitting direction of each radiation source according to the movement of the radiation emitting unit (100), thereby maintaining a penetration position identically before and after the movement of the radiation emitting unit.

The control unit controls the emitting direction of each radiation source, such that the emitting direction is towards the same position before and after the movement of the radiation emitting unit (100). That is, as the radiation emitting unit (100) moves, the second radiation source (120b) is arranged at the imaging position (7-1), and radiation is emitted in the emitting direction toward a penetration position, that is, the central part of the radiation detection unit (300). At this time, when the second radiation source (120b) is arranged at the imaging position (7-2) according to the movement of the radiation emitting unit (100), the penetration position of the second radiation source (120b) is changed as much as the moving distance of the radiation emitting unit (100). This feature is identically applied to the second radiation source (120a). Therefore, the control unit rotates each radiation source (120a, 120b) according to the movement of the radiation emitting unit (100) or changes a position of a focal point in the radiation sources (120a, 120b) to control the emitting direction of the radiation sources (120a, 120b), such that the emitting direction of the radiation sources (120a, 120b) according to the movement of the radiation emitting unit (100) is towards the central part of the radiation detection unit (300).

In addition, when the radiation sources (120) are sequenced along the shape of a straight line, a control is easy according to an interval between the radiation sources (120a, 120b) and the movement of the radiation emitting unit (100), but intervals from a penetration position, for example, the central part of the radiation detection unit (300) become different.

When each imaging position is arranged along the shape of a straight line and intervals from the central part of the radiation detection unit (300) are different, radiation emitted by the radiation sources from each imaging position may be incident onto the object (P) with different levels of strength. In this case, a radiation projected image acquired by radiation incident with different levels of strength may be image-processed and then calibrated. However, an additional image processing procedure may be omitted by controlling the radiation before acquiring the projected image.

Hereupon, the control unit may control a radiation emission amount of the radiation sources (120a, 120b) according to an interval between the imaging position and the penetration position, such that radiation emitted from each radiation sources (120) is incident onto the object (P) with uniform strength. That is, at the imaging position where the distance between the radiation source and the penetration position is relatively far, the control unit increases a radiation emission amount of the radiation source, and at the imaging position where the distance between the radiation source and the penetration position is relatively close, the control unit decreases a radiation emission amount of the radiation source so that radiation emitted from each radiation source may be incident onto the object (P) with uniform strength.

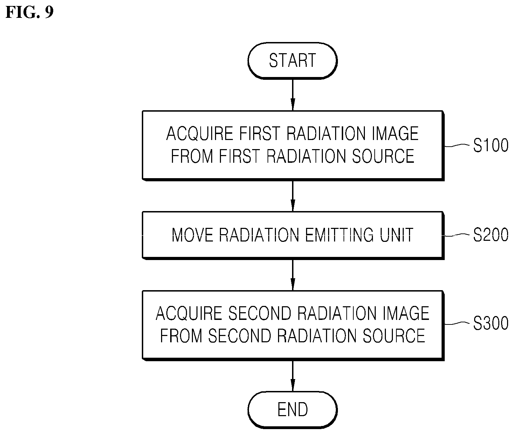

FIG. 9 is a drawing schematically illustrating a radiography method according to an embodiment of the present invention.

When referring to FIG. 9, the radiography method according to an embodiment of the present invention includes: a step of acquiring a first radiation image by activating the first radiation source arranged at one imaging position among a plurality of radiation sources provided in a radiation emitting unit (100) (S100); a step of moving the radiation emitting unit (100) (S200); and a step of acquiring a second radiation image by activating the second radiation source arranged at one imaging position among the plurality of radiation sources provided in the radiation emitting unit (100) (S300).

In the step (S100) of acquiring a first radiation image by activating the first radiation source, a first radiation image is acquired by activating the first radiation source arranged at one imaging position among a plurality of radiation sources that are provided in the radiation emitting unit (100) and include the first radiation source arranged at one imaging position and the second radiation source arranged at a position spaced apart from each imaging position.

That is, before the radiation emitting unit (100) moves, the first radiation source (120a) may be arranged at the imaging position (7-4) in a direction perpendicular to the radiation detection unit (30). In this case, before the radiation emitting unit (100) moves, the first radiation source (120a) is activated and captures a pre-shot image which is a radiation image for determining an imaging condition such as an exposure amount of radiation.

In addition, after a pre-shot image is captured or when a pre-shot image needs not to be captured, the second radiation source (120b) is arranged at the imaging position (7-1) and captures a first radiation projected image as the radiation emitting unit (100) moves in the sequenced direction of imaging positions. That is, the first radiation image acquired in the step (S100) of acquiring a first radiation image by activating the first radiation source may be a pre-shot image or a radiation projected image.

In the step (S200) of moving the radiation emitting unit, the radiation emitting unit (100) moves along imaging positions, that is, in the sequenced direction of radiation sources. Here, in the step (S200) of moving the radiation emitting unit, the radiation emitting unit moves along an arc when the radiation sources are sequenced in the shape of the arc, and moves along a straight line when the radiation sources (120) are sequenced in the shape of the straight line.

Here, as described above, the driving unit may move the radiation emitting unit (100) by using a motor, an electromagnet, or the like, the support unit having the radiation emitting unit (100) seated thereon is further included, and the driving unit may easily control the moving direction of the radiation emitting unit (100) on the support unit. In addition, in the step (S200) of moving the radiation emitting unit, the driving unit may change a moving speed of the radiation emitting unit (100) according to an interval between the radiation source and the imaging position. That is, in a continuous shot method of capturing a radiation projected image during the movement of the radiation emitting unit (100), the driving unit minimizes a motion blur phenomenon by reducing a moving speed of the radiation emitting unit (100) when one radiation source is adjacent to the imaging position within a predetermined interval by moving the radiation emitting unit (100). Moreover, when each radiation source is spaced apart from the imaging position by a predetermined interval or longer, a moving speed of the radiation emitting unit (100) may be changed to shorten an imaging time by increasing a moving speed of the radiation emitting unit (100).

Furthermore, as described above, the radiation sources (120a, 120b) are plurally provided in the radiation emitting unit (100), such that at least one thereof is arranged at one imaging position and at least one thereof is arranged at a position spaced apart from each imaging position, according to the movement of the radiation emitting unit (100). In this case, each radiation source (120a, 120b) may be provided in the radiation emitting unit (100) to have different intervals respectively from adjacent imaging positions in one direction, and in the step (S200) of moving the radiation emitting unit, the radiation emitting unit (100) may move in a distance shorter than intervals between the imaging positions.

In the step (S300) of acquiring a second radiation image by activating the second radiation source, the second radiation image is acquired by activating the second radiation source arranged at one imaging position among a plurality of radiation sources that are provided in the radiation emitting unit (100) and include the first radiation source arranged at a position spaced apart from each imaging position and the second radiation source arranged at one imaging position.

As described above, when the first radiation source (120a) captures the pre-shot image, the second radiation source (120b) may capture a first radiation projected image after the step of moving the radiation emitting unit (100) and when a pre-shot image is not captured, the first radiation source (120a) may capture a second radiation projected image by moving the radiation emitting unit (100) after the second radiation source (120b) captures the first radiation projected image.

The steps, that are, the step (S100) of acquiring the first radiation image, the step (S200) of moving the radiation emitting unit, and the step (S300) of acquiring the second radiation image, may be repeated until all radiation images are acquired at each imaging position.

That is, when the radiation emitting unit (100) moves along the sequenced direction of the imaging positions after the second radiation source (120b) captures the first radiation projected image, the first radiation source (120a) is arranged at the imaging position (7-5), and the second radiation source (120b) is arranged at a position spaced apart from the imaging positions. Here, the first radiation source (120a) is activated at the imaging position (7-5), and captures the second radiation projected image. Such a procedure may be repeated until all radiation projected images are acquired in each imaging position (7-1, 7-2, ..., 7-7).

In addition, the step (S100) of acquiring the first radiation image and the step (S200) of acquiring the second radiation image are performed during the movement of the radiation emitting unit (100), and it is thus possible to use a continuous shot method in which a radiation projected image is captured by activating each radiation source (120a, 120b) during the movement of the radiation emitting unit (100) and another radiation projected image is captured by moving the radiation sources to a next imaging position.

Also, when a plurality of radiation sources are sequenced in the shape of a straight line, a penetration position of the radiation sources may be changed according to the movement of the radiation emitting unit (100). Thus, the radiography method according to an embodiment of the present invention may further include a step of changing the emitting direction of each radiation source, such that the emitting direction of the radiation source according to the movement of the radiation source is towards the same penetration position as before the movement.

The step of changing the emitting direction of each radiation source may change the emitting direction by rotating each rotation source (120) or by changing a position of a focal point in the radiation sources, such that the emitting direction of the radiation sources after the movement matches the emitting direction of the radiation sources before the movement, and this step is simultaneously performed with a step of moving the radiation emitting unit (100) in the sequenced direction of the radiation sources, such that an additional time consumption according to a change in the emitting direction of the radiation sources may be prevented.

In addition, the radiography method according to an embodiment of the present invention may further include a step of rotating the radiation detection unit (300) between the step (S100) of acquiring the first radiation image and the step (S200) of acquiring the second radiation image. The step of rotating the radiation detection unit (300) may be performed before the step (S200) of moving the radiation emitting unit (100), during the step (S200) of moving the radiation emitting unit (100), or after the step (S200) of moving the radiation emitting unit (100). Here, as described above, the radiation detection unit (300) may be rotated to face each imaging position among a plurality of imaging positions, and in this case, radiation emitted from each imaging position may be maintained to be maximally emitted to the radiation detection unit (300).

By the first radiation image and the second radiation image acquired according to the above-described steps, all radiation projected images captured at an N number of positions, for example, seven imaging positions (7-1, 7-2, ..., 7-7), at each of imaging angle required to synthesize a three-dimensional image may be acquired. The radiation projected images at each of acquired imaging angle are synthesized by a reconstitution processing, such that a plurality of tomography images are generated. The reconstitution processing may be performed by using a filtered back projection (FBP) method. In such a calculation processing, a measured radiation projected image is filter-processed such that an image is back-projected, and the plurality of tomography images generated by the reconstitution processing may be displayed as a three-dimensional image corresponding to planes of different distances.

Likewise, according to the radiography apparatus and the radiography method using the same of embodiments of the present invention, since the radiation sources, of which at least one is arranged at one imaging position and at least one is arranged at a position spaced apart from each imaging position, captures the radiation projected image at the imaging positions according to the movement of the radiation emitting unit, the moving distance of the radiation emitting unit can be minimized, and a capturing time can thus be shortened.

In addition, since the radiation emitting unit, in which a plurality of radiation sources are provided, moves to acquire each radiation projected image at each of imaging position, the number of radiation sources can be reduced , and since a moving speed of the radiation emitting unit is reduced at the time of acquiring the radiation projected image, a motion blur phenomenon can be minimized.

Also, according to the radiography apparatus and the radiography method using the same of embodiments of the present invention, since a plurality of radiation sources provided in a radiation emitting unit is sequentially activated and captures a radiation projected image in various angles, each radiation projected image can be rapidly captured without considering a standby time to activate the radiation sources; therefore, a three-dimensional image of a high resolution can be acquired, and a lesion with respect to an object can be accurately diagnosed.

Although preferred embodiments of the present invention have been explained and illustrated above using specific terms, such terms are merely to clearly explain the present invention, and it would be obvious that various modifications and changes can be made to embodiments of the present invention and described terms without departing from the technical spirit and scope of the appended claims. These modified embodiments should not be understood to be individual from the spirit and scope of the present invention, but they should be understood to be included in the scope of claims of the present invention.

## Claims

1. A radiography apparatus comprising:
a radiation emitting unit for emitting radiation to an object;
a driving unit for moving the radiation emitting unit;
a radiation detection unit for detecting radiation emitted from each of a plurality of imaging positions provided at each of imaging angle with respect to the object, so as to acquire a plurality of radiation images; and
a plurality of radiation sources provided in the radiation emitting unit, such that, according to the movement of the radiation emitting unit, at least one thereof is arranged at one imaging position and at least one thereof is arranged at a position spaced apart from each imaging position.

2. The radiography apparatus of claim 1, wherein the radiation sources are provided in the radiation emitting unit to have different intervals respectively from the adjacent imaging positions in one direction.

3. The radiography apparatus of claim 1, wherein the radiation sources are provided in the radiation emitting unit such that an interval therebetween is greater than each interval between the imaging positions.

4. The radiography apparatus of claim 1, wherein the radiation sources are sequenced in one direction, and
the driving unit moves the radiation emitting unit along the sequenced direction of the radiation sources.

5. The radiography apparatus of claim 1, wherein the radiation sources are integrally moved with the radiation emitting unit while maintaining the interval therebetween.

6. The radiography apparatus of claim 1, wherein the radiation sources are sequentially activated according to the movement of the radiation emitting unit.

7. The radiography apparatus of claim 1, wherein the radiation detection unit acquires each radiation image during the movement of the radiation emitting unit.

8. The radiography apparatus of claim 1, wherein the driving unit changes a moving speed of the radiation emitting unit according to an interval between each radiation source and each imaging position.

9. The radiography apparatus of claim 1, further comprising a control unit for controlling an emitting direction of each radiation source according to the movement of the radiation emitting unit.

10. The radiography apparatus of claim 9, wherein the control unit controls the emitting direction of the radiation sources, such that the emitting direction of each radiation source is towards the same position according to the movement of the radiation emitting unit.

11. The radiography apparatus of any one of claims 1 to 9, wherein the radiation detection unit is provided to be rotatable according to the movement of the radiation emitting unit.

12. A radiography method comprising:
acquiring a first radiation image by activating a first radiation source arranged at one imaging position among a plurality of radiation sources provided in a radiation emitting unit;
moving the radiation emitting unit; and
acquiring a second radiation image by activating a second radiation source arranged at one imaging position among the plurality of radiation sources provided in the radiation emitting unit.

13. The radiography method of claim 12, wherein in the moving of the radiation emitting unit, the radiation emitting unit moves in a distance shorter than each interval between the imaging positions.

14. The radiography method of claim 12, wherein in the moving of the radiation emitting unit, a moving speed of the radiation emitting unit is changed according to an interval between each radiation source and each imaging position.

15. The radiography method of claim 12, wherein the acquiring of the first radiation image and the acquiring of the second radiation image are performed while the radiation emitting unit moves.

16. The radiography method of claim 12, further comprising changing an emitting direction of each radiation source, such that the emitting directions of the first radiation source and the second radiation source are towards the same position as a position before the radiation emitting unit moves.

17. The radiography method of claim 12, further comprising the rotating of a radiation detection unit between the acquiring of the first radiation image and the acquiring of the second radiation image.

18. The radiography method of claim 12, wherein the acquiring of the first radiation image, the moving of the radiation emitting unit, and the acquiring of the second radiation image are repeated until all radiation images are acquired at each imaging position.

19. The radiography method of any one of claims 12 to 17, wherein the first radiation image comprises a pre-shot image.
